(19) Europäisches Patentamt
European Patent Office
Office européen des brevets

(11) EP 3 447 496 B1

(12) EUROPEAN PATENT SPECIFICATION

(45) Date of publication and mention
of the grant of the patent:
16.10.2024 Bulletin 2024/42

(51) International Patent Classification (IPC):
A61K 49/00 (2006.01)   A61K 47/36 (2006.01)
A61K 9/00 (2006.01)

(21) Application number: 17786214.1

(22) Date of filing: 21.04.2017

(52) Cooperative Patent Classification (CPC):
A61K 49/0071; A61K 9/0024; A61K 47/36;
A61K 49/0034; A61K 49/0091; A61K 9/0019

(86) International application number:
PCT/KR2017/004302

(87) International publication number:
WO 2017/183946 (26.10.2017 Gazette 2017/43)

(54) **INJECTION COMPOSITION FOR LABELING LESION**

INJEKTIONSZUSAMMENSETZUNG ZUR LÄSIONSMARKIERUNG

COMPOSITION INJECTABLE POUR LE MARQUAGE DE LÉSION

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: 22.04.2016 KR 20160049425
20.04.2017 KR 20170050971

(43) Date of publication of application:
27.02.2019 Bulletin 2019/09

(73) Proprietor: National Cancer Center
Goyang-si, Gyeonggi-do 10408 (KR)

(72) Inventors:
• KIM, Seok Ki
Seoul 03095 (KR)
• PARK, Insoo
Seoul 07525 (KR)
• NOH, Jin Hee
Goyang-si
Gyeonggi-do 10372 (KR)

(74) Representative: Vossius & Partner
Patentanwälte Rechtsanwälte mbB
Siebertstrasse 3
81675 München (DE)

(56) References cited:
EP-A1- 2 940 474      WO-A1-2014/014956

KR-A- 20140 083 727      US-A1- 2008 147 038

• H. CHARLES MANNING: "World Molecular Imaging Congress 2015 - Final Program", 4 September 2015 (2015-09-04), XP055639723, Retrieved from the Internet <URL:http://www.wmis.org/wp-content/uploads/2015/09/WMIC2015_ProgramBook.pdf> [retrieved on 20191106]
• H. CHARLES MANNING: "Date stamp (Google) for "World Molecular Imaging Congress 2015 -Final Program" XP055639723", 4 September 2015 (2015-09-04), XP055639962, Retrieved from the Internet <URL:http://www.wmis.org/wp-content/uploads/2015/09/WMIC2015_ProgramBook.pdf> [retrieved on 20191107]
• KANEKO, M. ET AL.: "Positive Scintigraphy of Tumor by Means of Intra-arterial Injection of Radioiodinated Macroaggregated albumin (MAA", AMERICAN JOURNAL OF ROENTGENOLOGY, vol. 102, no. 1, January 1968 (1968-01-01), pages 81 - 87, XP055432577

- **BROUWER, O. R. ET AL.: "Comparing the Hybrid Fluorescent-radioactive Tracer Indocyanine Green-99mTc-nanocolloid with 99mTc-nanocolloid for Sentinel Node Identification: a Validation Study Using Lymphoscintigraphy and SPECT/CT", THE JOURNAL OF NUCLEAR MEDICINE, vol. 53, no. 7, 29 May 2012 (2012-05-29), pages 1034 - 1040, XP055432581**

**Description**

[Technical Field]

The present invention relates to an injection composition for labeling a lesion as defined in the claims and a method for providing information on a position of a lesion using the injection composition, as defined in the claims.

[Background Art]

Although various methods for anticancer treatment using anticancer drugs have been developed, removal of cancer cells through surgical methods remains the most widely used method of treatment.

However, surgical methods essentially require techniques for minimizing the range affected by surgery in order to improve a patient's health and well-being after the surgical operation. In particular, in order to achieve the goal of breast-conservation surgery for Korean women who typically have small breasts, the lesions that are surgically resected need to be smaller in Korean women than in the case of women from other countries.

Meanwhile, the range affected by surgery is determined in consideration of both the lesion and marginal regions around the lesion. When a surgeon cannot determine the exact location or extent of the lesion, the surgeon must set a wide marginal region around the lesion. This is because part of a tumor may remain in a resection margin of the lesion when the range of surgery is reduced without care.

However, there are not many methods that can be utilized for a surgeon to precisely identify a lesion in a real-time manner during actual clinical surgery. Although very precise diagnosis methods have been developed, because such diagnosis methods cannot be used during surgery, a surgeon depends mainly on tactile or visual senses during actual surgery, and lesions are often not clearly identified in such cases.

Particularly, smaller lesions are more difficult to identify. To achieve the objectives of minimally invasive surgery and preservation surgery, technology that shows a surgeon a lesion in real time during the surgery is needed.

In conventional surgeries for removing tumors, particularly breast cancer surgery, a position of a minute lesion in a patient is determined before surgery using ultrasonic waves, mammography, or magnetic resonance imaging, the predetermined position of the lesion is marked, and tissues related to the marked lesion are removed. Methods used to mark lesions include a method of drawing on the surface of the skin, a method of using a wire, a method of injecting a black pigment such as charcoal, and the like.

However, while the method of drawing on the surface of the skin with a pen to mark a position of the lesion is the easiest to use, it also has problems in that it has a very low degree of accuracy because the shape of the breast changes between diagnosis and the operation due to the extremely soft nature of breast tissue, and this method is unsatisfactory in regards to marking a position of the lesion on a skin surface when the lesion is located deep within the breast.

Further, in the method of piercing a breast lesion with a wire, it would be optimal to insert the wire to be perpendicular to the surface of the skin. However, such a method has problems in that the wire has to be inserted obliquely due to its potential influence on ultrasonic probing, a position of the wire may vary with movement of the breast, which results in lower accuracy than one would expect, the inserted wire may interfere with the surgery, and an additional procedure of resecting a site of the inserted wire is necessary. Finally, while the method of injecting a pigment such as charcoal has an advantage in that the injected pigment can bind to a lesion to mark the precise position of the lesion, it also has drawbacks in that a black pigment cannot be identified from outside of the breast when the lesion is located deep within the breast, and a surgical site may be contaminated with the pigment. The aforementioned drawbacks may also apply to surgical operations for removing cancerous tissues other than in cases of breast cancer.

Because it is difficult to precisely determine the range of surgery for surgically removing a lesion, particularly a cancerous lesion, using techniques that have been developed so far, the range of resection has to be expanded when a cancerous lesion is surgically removed, and tests for determining whether the lesion has been properly removed also have to be conducted after surgery.

To solve these problems, Korean Registered Patent No. 10-1552138 discloses a composition for labeling a cancerous lesion, which includes a complex in which a dye for staining biological tissues, a radioactive isotope or a combination thereof is bound to macroaggregated albumin. In this case, the composition allows a marker to be effectively absorbed onto a cancerous lesion to mark the exact position of the lesion, and allows the pigment to be traced in real time so that the range of the lesion to be removed can be confirmed.

Meanwhile, while the complex in the form of an aqueous solution disclosed in Korean Registered Patent No. 10-1552138 is favorable as a marker for a surgical operation because the complex does not diffuse into tissues in a single pigment form, in actual application, it has a drawback in that a predetermined amount of the complex cannot be injected because it precipitates in the injection preparation.

Also, too much or too little of the complex in the form of an aqueous solution disclosed in Korean Registered Patent No. 10-1552138 may be injected due to its low viscosity. Therefore, when a practitioner has little experience with injections or a situation in a clinic makes administering injections difficult, a predetermined amount of the macro aggregated albumin cannot be injected. When the complex in the injection preparation settles in the syringe, the complex is injected in the form of a lump, and irregularities are sometimes observed in the marked area. Also, when the complex is injected with a large amount or when tissues into which the complex is injected are tough to penetrate (scar tissue or muscle tissue), the injected solution tends to spread widely along loose tissues of membranes only between different tissues or regions of muscle.

That is, the injected solution may spread widely into muscles along the muscle fascia, and may extend into scar tissues along the grains of fibrous tissues in the scar tissues. To prevent this, a small amount of the injected solution should be injected using a relatively thick needle, but this method has a problem in that it is very inconvenient in terms of practice.

Accordingly, there is a need for an injection composition capable of solving the problems of clogging of an injection needle due to a lumped complex within an injection, inability to inject a predetermined amount of the complex, and rapid spreading of the complex into the surrounding tissues.

[Disclosure]

[Technical Problem]

To solve the above problems, it is an object of the present invention to provide an injection composition for labeling a lesion, which is capable of solving the problem of the rapid settling of the complex by addition of a composition which may regulate viscosity in the injection composition.

In particular, it is an object of the present invention to provide an injection composition for labeling a lesion that is capable of solving the problem of the rapid spread of the complex into surrounding tissues, in which the problem of rapid settling of the complex is also solved and thus a predetermined amount of the complex can be injected.

It is another object of the present invention to provide a method for providing information on a position of a lesion using the aforementioned injection composition for labeling a lesion.

[Technical Solution]

The invention is defined by the claims. Any subject-matter falling outside the scope of the claims is provided for information purposes only. Any references in the description to methods of treatment, diagnosis or surgery refer to the compounds, pharmaceutical compositions and medicaments of the present invention for use in a method for treatment, diagnosis or surgery of the human or animal body.

To achieve the objects of the present invention, according to an aspect of the present invention, an injection composition for labeling a lesion is provided, which includes:

a first composition including a complex containing an active ingredient and having an average density of 1.1 to 1.4 g/mL, the active ingredient being a dye for staining biological tissues, a radioactive isotope or a combination thereof bound to macroaggregated albumin (MAA); and a second composition being hyaluronic acid (HA), the HA having an average molecular weight of 0.5 to 3.0 MDa, wherein the HA is included at 0.2 to 1 % (w/v) with respect to the total amount of the injection composition.

According to another aspect of the present invention, a method for providing information on a position of a lesion to which the above injection composition has been administered is provided, which includes:

determining the position of the lesion through a signal of an active ingredient generated in the subject.

[Advantageous Effects]

An injection composition for labeling a lesion according to the present invention includes a first composition including a complex containing an active ingredient and a second composition including hyaluronic acid (HA), which makes it possible to solve the problem of rapid settling of the complex.

In particular, the injection composition for labeling a lesion according to the present invention can have an effect of solving the problem of rapid settling of the complex during preparation of the injection so that a predetermined amount of the complex can be injected, and an effect of providing clinically convenient and stable use of the complex by solving the problems of injection of an excessive amount of the complex and the rapid spread of the complex to its surroundings.

Also, when a predetermined amount of a biocompatible viscous material such as hyaluronic acid or collagen is added to the injection composition of the present invention to inject an equivalent amount of the viscous material into various tissues such as muscle tissue, breast tissue, subcutaneous tissue, skin tissue, and the like, the injection composition of the present invention can have an effect of allowing a predetermined amount of the complex to be injected

EP 3 447 496 B1

much more effectively, compared to when the viscous material is not included, and can also have an effect of greatly reducing spreading of the complex even when the injection composition is injected rapidly, compared to when the viscous material is not included.

[Description of Drawings]

FIG. 1 is a graph of a linear regression equation for estimating the density of ICG-MAA.
FIG. 2 is a graph illustrating a change in near-infrared fluorescence signal intensity of an ICG-MAA complex according to changes in concentrations of ICG and MAA.
FIG. 3 is an image illustrating a comparison between two types of MAA having different HA concentrations ((A) 0.1% HA and (B) 0.5% HA).
FIG. 4 shows a bright-field image of an MAA aqueous solution in the presence/absence of HA.
FIG. 5 shows fluorescence signals measured of 5 $\mu$M ICG at various MAA concentrations.
FIG. 6 shows near-infrared fluorescent (NIRF) images of chicken breasts and gizzards photographed before and after incision after injecting the complex into the chicken breasts and gizzards.
FIG. 7 shows a bright-field and NIRF image of a section of an incised chicken breast observed after the complex was injected along an injection needle pathway.
FIG. 8 shows the testing of the applicability of an ICG-MAA-HA mixture in gastric cancer surgery using an endoscopic catheter.
FIG. 9 is a diagram showing the results of measuring fluorescence signals based on the presence/absence of hyaluronic acid ((A) hyaluronic acid is not added, and (B) 0.1% hyaluronic acid is added).

[Best Mode]

Specific embodiments of the present invention are illustrated in the drawings and described in detail in the detailed description.
It will be further understood that the terms "comprises," "comprising," "includes," "including," "has" and/or "having," when used herein, specify the presence of stated features, integers, steps, operations, elements, components and/or groups thereof, but do not preclude the presence or addition of one or more other features, integers, steps, operations, elements, components and/or groups thereof.
The present invention is directed to an injection composition for labeling a lesion, and, more particularly, to an injection composition for labeling a lesion, which is capable of solving the problem of rapid settling of a complex by addition of a composition capable of regulating the viscosity of the injection composition to the injection composition.
In particular, the injection composition for labeling a lesion according to the present invention may solve the problem of rapid settling of the complex by addition of a composition capable of regulating viscosity in the injection composition, and thus may have an effect of allowing a predetermined amount of the complex to be injected.
Hereinafter, the present invention will be described in detail.
According to one exemplary embodiment of the present invention, an injection composition for labeling a lesion is provided, which includes:
a first composition including a complex containing an active ingredient and having an average density of 1.1 to 1.4 g/mL, the active ingredient being a dye for staining biological tissues, a radioactive isotope or a combination thereof bound to macroaggregated albumin (MAA); and a second composition being hyaluronic acid (HA), the HA having an average molecular weight of 0.5 to 3.0 MDa, wherein the HA is included at 0.2 to 1 % (w/v) with respect to the total amount of the injection composition.
More specifically, because a complex in which an active ingredient is bound to macro aggregated albumin has relatively high density, that is, an average density of 1.1 to 1.4 g/mL, the complex has a drawback in that the complex settles in a syringe during preparation of an injection.
Therefore, the present invention is designed to solve the problem of the rapid settling of the complex during preparation of the injection, and is characterized by addition of a second composition being hyaluronic acid (HA), the HA having an average molecular weight of 0.5 to 3.0MDa to the aforementioned first composition to regulate the viscosity of the injection composition.
The first composition of the present invention will first be described in detail below.
According to one exemplary embodiment of the present invention, the first composition includes a composition in which an active ingredient is bound to macroaggregated albumin (MAA).
In the present invention, the term "macroaggregated albumin (MAA)" refers to a proteinaceous particle having a diameter of 10 to 50 $\mu$m, which is prepared by heating human serum albumin so that the human serum albumin

5

coagulates.

Meanwhile, the structure and physical properties of the MAA may differ from those of human serum albumin, which has a diameter of less than 10 nm. Because the MAA may have a characteristic of staying in pulmonary capillaries having a diameter of approximately 8 $\mu$m when intravenously injected, which may cause a microembolus, MAA labeled with a radioactive isotope has been used for pulmonary scintigrams (for diagnosis of pulmonary blood flow abnormalities such as pulmonary embolisms, pulmonary thrombosis, aortitis syndrome, pneumonia, lung cancer, and the like, or diagnosis of right and left shunts or pulmonary venous hypertension), vein scans (for diagnosis of central venous blood at a site to be scanned), artery scans (for diagnosis of peripheral arterial blood flow abnormalities such as Paget's disease and the like), or the like by utilizing the aforementioned characteristic. The MAA of the present invention may be used as a mediator for binding a labeling material with a cancerous lesion tissue when the MAA is injected into the cancerous lesion tissue.

In addition, the MAA of the present invention may be synthesized using recombinant HSA and also non-autologous HSA. Also, MAA that is commercially available may be purchased and used. The MAA of the present invention may be used as a mediator for binding a labeling material with cancerous lesion tissue when the MAA is injected into the cancerous lesion tissue. In this case, the mediator may serve to adsorb a labeling material and prevent the labeling material from spreading into the cancerous lesion tissue.

Further, according to one exemplary embodiment of the present invention, the term "active ingredient" refers to a component that raises the energy of molecules, atomic ions, and the like by absorption, discharge, and particle beam bombardment of radiant energy so that it is liable to cause a chemical reaction. In this case, the active ingredient is a dye for staining biological tissues, a radioactive isotope, or a combination thereof.

According to one exemplary embodiment of the present invention, the dye for staining biological tissues may be a visually detectable dye or a fluorescent dye.

The visually detectable dye may be selected from the group consisting of neutral red, Nile blue, Bismarck brown, lithium carmine, trypan blue, Janus Green, methyl violet, O-lamin, Malachite green, safranin, eosin, Congo red, erythrosine, nigrosin, Alcian blue haematoxylin, aniline blue, light green, and a combination thereof.

According to one exemplary embodiment of the present invention, the fluorescent dye may be a near-infrared fluorescent dye, and the near-infrared fluorescent dye may be indocyanine green (ICG), but the present invention is not limited thereto.

In the present invention, the term "dye for staining biological tissues" refers to a substance that binds to biological tissue to mark a position of the bound tissue so that a position of the labeled tissue can be determined with the naked eye or a detection tool. For the purposes of the present invention, the dye for staining biological tissues may be a labeling material that may bind to a cancer tissue so that the labeling material can be used for the purpose of labeling a site at which cancer has developed. Preferably, a visually detectable dye, a fluorescent dye capable of emitting fluorescence at a binding site so that the dye can be detected using an apparatus such as a fluorescence camera, and the like may be used alone or in combination with each other, but the present invention is not particularly limited thereto.

In the present invention, the term "visually detectable dye" refers to a type of pigment that allows a labeling material bound to biological tissue to emit a color within a visible color spectrum so that a position of the labeled tissue can be determined with the naked eye. For the purposes of the present invention, when the visually detectable dye is injected into a site at which cancer has developed for the purpose of removing cancer using a surgical method, the visually detectable dye may serve to improve a success rate of cancer surgery because it allows for the clear identification of a cancerous lesion to be resected. The visually detectable labeling material preferably includes neutral red, Nile blue, Bismarck brown, lithium carmine, trypan blue, Janus green, methyl violet, O-lamin, Malachite green, safranin, eosin, Congo red, erythrosine, nigrosin, Alcian blue haematoxylin, aniline blue, light green, or the like, which may be used alone or in combination. However, the visually detectable labeling material is not particularly limited as long as it can achieve the goal of identifying cancerous lesion tissue.

In the present invention, the term "fluorescent dye" refers to an organic compound that emits fluorescence. In this case, the fluorescent dye may absorb light with certain wavelengths to form an excited state and maximize the penetration distance of light and may minimize error signals caused by moisture. Preferably, the organic compound may be a near-infrared fluorescent dye that is an organic compound emitting fluorescence with a near-infrared wavelength of 700 nm to 3,000 nm, preferably 750 nm to 900 nm. The fluorescence of near-infrared wavelengths emitted from the near-infrared fluorescent dye may be photographed in the form of images or monitored in real time using an apparatus such as a fluorescence camera, a fluorescent sensing probe (PCT/KR201 1/009271), or the like. In the present invention, because the fluorescence of near-infrared wavelengths is absorbed *in vivo* at a relatively small dose, in contrast to other wavelength ranges, near-infrared light emitted from a site located relatively deep within a biological tissue may also be detected from outside of the biological tissue. For the purposes of the present invention, when the near-infrared fluorescent dye is injected into a site at which cancer has developed to remove cancer using a surgical method, the near-infrared fluorescent dye may serve to improve a success rate of cancer surgery because it allows for the clear identification of a lesion site of cancer before resection. In particular, unlike the visually detectable dye, the near-infrared fluorescent dye may promote rapid and accurate cancer surgery because the near-infrared fluorescent dye may be used to externally detect a position of a lesion before a tissue incision is made to directly identify the lesion. Indocyanine green and the like may be preferably used as the near-infrared fluorescent dye. At this time, any near-infrared fluorescent dye that can be used in the human body may be included in the scope of the present invention.

The complex in which the near-infrared fluorescent dye is bound to MAA may have a high probability related to finding a minute lesion and improve a degree of accuracy in lesion resection because the complex has an advantage in that the complex has excellent stability and accuracy related to fluorescence signals to be detected, compared to the complexes in which the near-infrared fluorescent dye is bound to other materials known to accumulate in tumors.

In the present invention, the term "indocyanine green (ICG)" refers to a fluorescent imaging dye emitting fluorescence in a near-infrared region, which is widely used in the field of biology or medicine. In this case, it is desirable that the ICG be clinically applied as a fluorescent dye that may be used for the human body because the ICG is decomposed and disappears or is excreted in the urine or feces within an hour after the ICG is injected into the human body. In fact, various theses have reported cases in which indocyanine green was used for application in the human body. One example reported the safe clinical use of indocyanine green in 18 breast cancer patients (T. Kitai, et al., Breast Cancer, 12:211-215, 2005). In addition, adsorptive binding of the near-infrared fluorescent dye may be achieved by mixing the near-infrared fluorescent dye with the MAA of the present invention.

According to one exemplary embodiment of the present invention, it was confirmed that a mixing ratio suitable for preparing a complex exhibiting a high level of near-infrared fluorescence signals upon the preparation of a complex in which ICG is bound to MAA (ICG-MAA) is 3.9 $\mu$M ICG with respect to 0.23 mg/mL MAA, 6.5 $\mu$M ICG with respect to 2.3 mg/mL MAA, and 6.5 $\mu$M ICG with respect to 11.5 mg/mL MAA. Because the concentrations of MAA and ICG vary due to *in vivo* diffusion when MAA and ICG are injected *in vivo*, it is impossible to determine the exact concentrations of MAA and ICG at the time of injection. However, it was confirmed that the complex has the highest fluorescence value when injected at 6.5 $\mu$M ICG with respect to 2 to 4 mg/mL MAA, which is easily injected in an experimental aspect. Therefore, 6.5 $\mu$M ICG may be preferably used with respect to 2 mg/mL MAA.

In the present invention, the term "radioactive isotope" refers to an element that has the same atomic number but a different atomic weight and may emit radiation. In this case, the radioactive isotope is often used as an important marker for conventionally diagnosing diseases as a result of emitting gamma rays or other subatomic particles to make use of the radioactive decay characteristics of the radioactive isotope. For the purposes of the present invention, when the radioactive isotope is injected into a site at which cancer has developed in tissue at a depth at which the fluorescence emitted from the near-infrared fluorescent dye cannot be detected, to remove cancer using a surgical method, the radioactive isotope may serve to improve a success rate of cancer surgery because it allows the clear identification of a lesion site of cancer before resection. The radioactive isotope is not particularly limited as long as it has a property of being able to be labeled with MAA capable of binding to a lesion of cancer. However, the radioactive isotope may preferably be H-3, C-14, P-32, S-35, Cl-36, Cr-51, Co-57, Co-58, Cu-64, Fe-59, Y-90, I-124, 1-125, Re-186, I-131, Tc-99m, Mo-99, P-32, CR-51, Ca-45, Ca-68, or the like, and more preferably 1-124, 1-125, 1-131, Cu-64, Tc-99m, Mo-99, CR-51, Ca-45, Ca-68, or the like, all of which are used for medical purposes. Most preferably, Tc-99m may be used as the radioactive isotope.

In the present invention, the term "Tc-99m" refers to a radioactive isotope of technetium (Tc) that has been widely used for medical studies because it has a short half-life of 6 hours, produces gamma rays that can be used for imaging, requires a very low radiation dose, exhibits excellent tissue permeability, and does not cause an allergic reaction as is caused by some pigments.

In the present invention, the term "subject" refers to a living organism which may have a lesion due to the onset of cancer and to which a complex or composition for labeling a cancerous lesion according to the present invention may be administered.

When the injection composition for labeling a lesion provided in the present invention is administered *in vivo* into cancerous lesion tissue, the administered injection composition is bound to the cancerous lesion to mark a position of the lesion by means of color, near-infrared fluorescence, radioactivity, or a combination thereof. Also, because the position, size and the like of the cancerous lesion may be detected in real time during surgery by detecting the label, accuracy may be improved and excessive loss of healthy tissue may be prevented when the cancerous lesion is removed through a surgical operation.

In addition, because the complex included in the injection composition of the present invention may remain in the *in vivo* cancerous lesion for a long time, in contrast to the complexes in which the dye for staining biological tissues is bound to other material, a degree of accuracy related to resection of the cancerous lesion by surgical operation as well as surgical resection of the cancerous lesion may be readily determined. For example, after a position of a minute lesion is determined before surgery using ultrasonic waves, and the like, the complex of the present invention may be injected into a lesion site, and the lesion site may be stably and accurately determined during surgery within hours.

According to one exemplary embodiment of the present invention, the MAA is preferably used at a concentration of 1 to 8 mg/mL with respect to a buffer, but the present invention is not limited thereto.

According to one exemplary embodiment of the present invention, the ICG is preferably used at 4 to 250 μM with respect to 1 to 8 mg/mL MAA. In consideration of the solubility of MAA, 6.5 μM ICG may be more preferably used with respect to 2 to 4 mg/mL MAA. However, it is possible to properly adjust the concentrations of MAA and ICG upon *in vivo* injection within the ranges described in the examples to exhibit the maximum fluorescence intensity according to the conditions.

According to one exemplary embodiment of the present invention, the radioactive isotope may be selected from the group consisting of H-3, C-14, P-32, S-35, Cl-36, Cr-51, Co-57, Co-58, Cu-64, Fe-59, Y-90, 1-124, I-125, Re-186, 1-131, Tc-99m, Mo-99, P-32, CR-51, Ca-45, Ca-68, and a combination thereof.

Next, the second composition according to one exemplary embodiment of the present invention will be described in detail.

The second composition is characterized by having a molecular weight of 0.5 to 3.0 MDa and including hyaluronic acid (HA)

In particular, because the second composition has a predetermined viscosity, the second composition may be added to the injection composition for labeling a lesion to solve the problem of rapid settling of the complex included in the first composition, and thus has an effect of allowing a predetermined amount of the complex to be injected.

The molecular weight of the second composition is in a range of 0.5 to 3.0 MDa, or in a range of 1.0 to 2.0 MDa. Because the second composition has a relatively low molecular weight and a predetermined viscosity, the second composition may solve the problem of rapid settling of the complex included in the first composition.

Here, the molecular weight unit "Da" is a unit representing mass. In this case, 1/16 of the mass of an oxygen atom may be referred to as one dalton.

In addition, the second composition may have a viscosity of 5 to 1,500 cps at room temperature, and may have a viscosity of 100 to 900 cps, preferably a viscosity of 100 to 350 cps, at room temperature.

More specifically, when the viscosity of the second composition is less than 5 cps at room temperature, it is impossible to solve the problem of rapid settling of the complex included in the first composition due to low viscosity. On the other hand, when the viscosity of the second composition is greater than 1,500 cps, it may be difficult to inject the injection composition due to very high viscosity, and thus a high amount of pressure needs to be applied when the injection composition is injected into tissue.

According to the present invention, the second composition includes a biocompatible viscous material, and the biocompatible viscous material is hyaluronic acid (HA).

According to the present invention, the second composition is added at an amount of 0.2% (w/v) to 1% (w/v), based on the total weight of the injection composition.

More specifically, when the second composition is included at an amount of less than 0.2% (w/v) based on the total weight of the injection composition, it may be difficult to inject a predetermined amount of the complex into tissue because the complex settles rapidly due to a short suspensibility maintenance time of the injection composition. Particularly, the problem of injecting an excessive amount of the complex or the complex spreading into the surrounding tissue too quickly when injected into the tissues arise.

Also, when the content of the second composition is greater than 1% (w/v) based on the total weight of the injection composition, an increase in viscosity may be caused due to the degree of concentration of the second composition, and thus a high amount of pressure may need to be applied during injection, which makes it difficult to inject the injection composition by hand.

In one aspect, the injection composition may be added at 0.2% (w/v) to 1% (w/v) in a state in which the injection composition is loaded in a syringe including an 18 to 26G needle. In another aspect, the injection composition may be included at 0.2 to 0.5% (w/v) when using an endoscopic catheter.

Meanwhile, one exemplary embodiment of the present invention is characterized in that an injection composition for labeling a lesion which satisfies the following Mathematical Expression 1 when measured at room temperature is provided.

[Mathematical Expression 1]

$$|T_2 - T_1| < 15\%$$

wherein $T_1$ represents transmittance (%) at 550 nm when measured at the one quarter point of the height of a transparent container having a size of $1 \times 1 \times 3$ cm$^3$ in a state in which the prepared injection composition is loaded in the transparent container, and

$T_2$ represents transmittance (%) at 550 nm when measured at the one quarter point of the height of a transparent container having a size of $1 \times 1 \times 3$ cm$^3$ after being allowed to stand for 120 minutes in a state in which the prepared injection composition is loaded in the transparent container.

At this time, transmittance may refer to a degree of passage of light through a material layer. At this time, when the intensity of light incident on the material layer is $I_1$ and the intensity of light emitted through the material layer is $I_2$, transmittance may be considered to be $T = I_2I_1$.

In addition, the transparent container may have a size of $1\times1\times3$ cm$^3$ (width$\times$length$\times$height), but the present invention is not limited thereto.

Also, the term "one quarter point of the height of the transparent container" may refer to a point corresponding to one quarter of the container measured from the bottom thereof.

By way of one example, the transmittance ($T_1$) at 550 nm when measured at the one quarter point of the container in a state in which 0.4% (w/v) hyaluronic acid is added and the prepared injection composition is loaded in the transparent container having a size of $1\times1\times3$ cm$^3$ may be 0.4%. The transmittance ($T_2$) at 550 nm when measured at the one quarter point of the container after being allowed to stand for 120 minutes in a state in which the prepared injection composition is loaded in the transparent container having a size of $1\times1\times3$ cm$^3$ may be 78.71%.

That is, the suspensibility maintenance time may satisfy $|T_2\text{-}T_1| < 15\%$ because the suspensibility maintenance time is extended by adding a second component capable of regulating viscosity in the injection composition.

Further, in one exemplary embodiment of the present invention, an injection composition for labeling a lesion which satisfies the following Mathematical Expression 2 when measured at room temperature may be provided.

$$[\text{Mathematical Expression 2}]$$

$$|F_2 - F_1| < 20\ \text{gf}$$

wherein $F_1$ represents a gliding force measured at the start of injection in a state in which the prepared injection composition is loaded in a syringe with a 26G needle, and

$F_2$ represents a gliding force measured at the start of injection when injected after being maintained for 120 minutes in a state in which the prepared injection composition is loaded in a syringe with a 26G needle.

Here, the unit "gf" of the gliding force refers to the magnitude of force that may be referred to as gravitational force. Here, one gravitational force (g) represents 0.0098 N.

In addition, the "gliding force" refers to a gliding force that is exerted on a finger when the composition is injected through a syringe in a state in which the composition is loaded in a syringe.

Meanwhile, the 26 gauge (G) syringe may refer to a syringe with a needle having an inner diameter of 0.241 mm.

As a specific aspect, the gliding force measured according to Mathematical Expression 2 may be less than or equal to 5 gf. That is, $|F_2\text{-}F_1|$ may be less than or equal to 5 gf.

Particularly, a small numerical value for $|F_2\text{-}F_1|$ indicates a small difference in pressure between a starting point of injection after the injection composition is allowed to stand for a predetermined time and an intermediate point of injection, indicating that the suspensibility of the injection composition is maintained even when the injection composition is allowed to stand for 120 minutes, particularly that the settling rate of the complex in the injection composition has slowed down.

In addition, $F_2$ is characterized by having a gliding force of 120 to 165 gf. More specifically, $F_2$ may be in a range of 120 to 165 gf when the second composition is included at 0.2% (w/v) to 1% (w/v), based on the total weight of the injection composition.

That is, the injection composition of the present invention may have an effect of allowing a predetermined amount of a complex to be injected because the problem of rapid settling of the complex during injection preparation is solved by adding a predetermined amount of the biocompatible viscous material such as hyaluronic acid or collagen to the injection composition, and may also solve the problem of injecting an excessive amount of the complex and the problem of the complex spreading into the surrounding tissues. Also, the injection composition of the present invention may have an effect of greatly reducing spreading of the complex through the addition of a predetermined amount of the biocompatible viscous material such as hyaluronic acid or collagen to the injection composition when the same amount of the injection composition is injected into various tissues such as muscle tissue, breast tissue, subcutaneous tissue, skin tissue, and the like, compared to when the viscous material is not included. Even when the injection composition is injected rapidly, the injection composition of the present invention may have an effect of greatly reducing spreading of the complex, compared to when the viscous material is not included.

According to one exemplary embodiment of the present invention, the lesion may be a cancerous lesion, but the present invention is not limited thereto.

According to one exemplary embodiment of the present invention, the cancer may be a solid cancer selected from the group consisting of prostate cancer, breast cancer, uterine cancer, skin cancer, cervical cancer, lung cancer, brain tumors, gastrointestinal tumors, liver cancer, soft tissue sarcoma, lymphoma, and a combination thereof.

According to one exemplary embodiment of the present invention, the injection composition according to the present invention may be used to determine a size and position of the cancerous lesion tissue in real time during the surgery for cancer removal.

Also, the present invention is directed to a method for providing information on a position of a lesion to which the injection composition according to the present invention has been administered, which includes:
determining a position of the lesion using a signal of an active ingredient generated in the subject.

[Mode for Invention]

Hereinafter, the present invention will be described in further detail with reference to examples and experimental examples thereof. However, it will be apparent to persons having ordinary skill in the art that the following examples are merely given to describe the present invention more fully, and are not intended to limit the scope of the present invention.

## <PREPARATION EXAMPLES>

### Preparation Example 1: Preparation of macroaggregated albumin (MAA)

To prepare macroaggregated albumin (MAA), 10 mL of 2% human serum albumin (SK Plasma) diluted with a 0.1 M acetate buffer (pH 5.4, Sigma-Aldrich, Korea) was mixed with 50 mg of tin chloride (Sigma-Aldrich, Korea), and the resulting mixture was strongly stirred at room temperature for 10 minutes, and then reacted while stirring at 70 °C for another 20 minutes.

Next, to prevent additional coagulation after the reaction was completed, a reaction product was put on ice for rapid cooling.

Then, 0.35 mL of 20% human serum albumin (70 mg in the case of human serum) was added to the cooled reaction product, and then stirred at room temperature for 10 minutes. Thereafter, a reaction product was divided into glass vials at contents of 1, 2, 4, and 8 mg with respect to the MAA, and then freeze-dried to prepare freeze-dried MAA kits.

The MAA kits ultimately included the freeze-dried MAA at contents of 1, 2, 4, and 8 mg.

As described above, the acetate buffer was used during a preparation process when the MAA was prepared, and the freeze-dried MAA kits were used after the MAA kits were dissolved in water for injection or water for injection including HA.

### Preparation Example 2: Preparation of marker based on MAA to which indocyanine green (ICG) is bound

### Preparation Example 2-1: Determination of mixing ratio of ICG and MAA

To prepare an MAA-based marker emitting near-infrared fluorescence, indocyanine green (ICG, Jeil Pharmaceutical Co., Ltd., Diagnogreen Injection) emitting near-infrared fluorescence was bound to the MAA prepared in Example 1 to prepare a complex (ICG-MAA).

In this case, to determine the mixing ratio of ICG and MAA capable of emitting the strongest near-infrared fluorescence, 1.3 to 1,032 $\mu$M of ICG and 0 to 11.5 mg/mL of MAA were reacted at various ratios to prepare the corresponding ICG-MAA complexes.

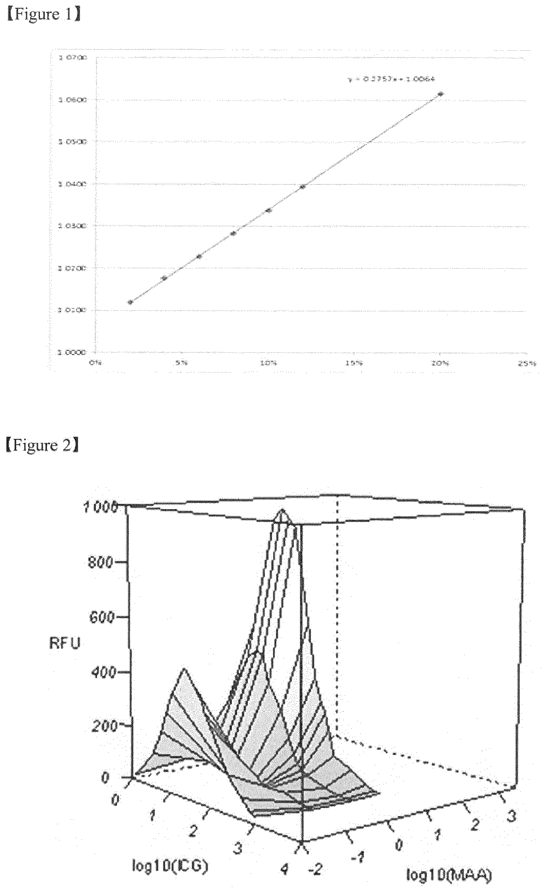

Then, the signal intensities of near-infrared fluorescence emitted from the prepared ICG-MAA complexes were measured (Table 1 and FIG. 2).

FIG. 2 is a graph illustrating a change in near-infrared fluorescence signal intensity of the ICG-MAA complex according to changes in concentrations of ICG and MAA.

[Table 1]

| ICG ($\mu$N) | MAA (mg/mL) | | | |
|---|---|---|---|---|
| | 0 | 0.23 | 2.3 | 11.5 |
| 1.3 | 18 | 42 | 238 | 530 |
| 3.9 | 120 | 52 | 424 | 931 |
| 6.5 | 212 | 38 | 456 | 979 |
| 9.0 | 289 | 32 | 444 | 942 |
| 12.9 | 363 | 27 | 342 | 915 |
| 25.8 | 466 | 12 | 255 | 563 |

(continued)

| ICG ($\mu$N) | MAA (mg/mL) | | | |
|---|---|---|---|---|
| 38.7 | 425 | 8 | 162 | 366 |
| 51.6 | 399 | 7 | 101 | 280 |
| 64.5 | 374 | 13 | 75 | 244 |
| 77.4 | 332 | 16 | 55 | 182 |
| 103 | 289 | 23 | 39 | 94 |
| 258 | 139 | 30 | 16 | 60 |
| 516 | 71 | 13 | 2 | 20 |
| 774 | 39 | 6 | 2 | 9 |
| 1032 | 30 | 6 | 1 | 4 |

Referring to Table 1 and FIG. 2, it was confirmed that 25.8 $\mu$M ICG had the highest value of near-infrared fluorescence signal intensity when MAA was not treated, and that 3.9 $\mu$M ICG had the highest value of near-infrared fluorescence signal intensity when 0.23 mg/mL of MAA was treated.

In addition, it can be seen that 6.5 $\mu$M ICG had the highest value of near-infrared fluorescence signal intensity when 2.3 mg/mL of MAA was treated, and that 7.5 $\mu$M ICG had the highest value of near-infrared fluorescence signal intensity when 11.5 mg/mL of MAA was treated.

Meanwhile, because the concentrations of MAA and ICG were varied due to *in vivo* diffusion when MAA and ICG were injected *in vivo*, it was impossible to determine the exact concentrations of MAA and ICG at the time of injection. However, it was confirmed through experiments that the complexes had the highest fluorescence value when 6.5 $\mu$M ICG with respect to 2 mg/mL MAA was injected.

**Preparation Example 2-2: Preparation of ICG-MAA complex in which ICG is bound**

ICG-MAA complexes in which the concentrations of MAA were 1, 2, 4, and 8 mg/mL were prepared. The reactions for preparing the ICG-MAA complexes were carried out at room temperature.

More specifically, 0.5 mL of 13 $\mu$M ICG was added to each of the 1, 2, 4, and 8 mg MAA freeze-dried kits, and reacted while gently stirring for approximately one minute. Thereafter, 0.5 mL of distilled water was added to each of the MAA freeze-dried kits, and gently stirred for approximately five minute to prepare 6.5 $\mu$M ICG-MAA complexes having different MAA concentrations.

**<EXAMPLES>**

**Example 1: Preparation of ICG-MAA-HA**

ICG-MAA-HA injection compositions including MMA at concentrations of 1, 2, 4, and 8 mg/mL, 6.5 $\mu$M ICG, and hyaluronic acid (HA; Shandong Focuschem Biotech Co.) at a concentration of 0.5% were prepared.

More specifically, 0.5 mL of 13 $\mu$M ICG was respectively added to the 1, 2, 4, and 8 mg MAA freeze-dried kits, and reacted while gently stirring for approximately one minute. Thereafter, 0.5 mL of each of 0.2, 0.4, 0.6, 0.8, 1, 2, 4, and 8% (w/v) HA (1,448 kDa) dissolved in advance was added thereto, and then homogenously mixed while strongly stirring for approximately 5 minutes.

As a result, a total of 32 ICG-MAA-HA injection compositions including HA at concentrations of 0.1 , 0.2, 0.3, 0.4, 0.5, 1, 2, and 4% (w/v), ICG at a concentration of 6.5 $\mu$M, and MAA at concentrations of 1, 2, 4, and 8 mg/mL were prepared.

For reference, a process of preparing ICG-MAA-HA was carried out at room temperature.

**<EXPERIMENTAL EXAMPLES>**

**Experimental Example 1: Measurement of densities of ICG-MAA complexes**

The densities of the ICG-MAA complexes were measured.

Because the ICG-MAA complexes were present in the form actually used in an aqueous solution phase, it was difficult to directly measure the densities of the complexes without causing damage to the forms of biological materials, and the like. Therefore, the densities of the ICG-MAA complexes were measured indirectly with reference to a change in density according to concentration.

The density was measured using the prepared ICG-MAA complex including MMA at a concentration of 2mg/mL and 6.5 μM ICG, and the ICG-MAA densities were measured using the ICG-MAA complexes at concentrations of 20 mg/mL to 200 mg/mL (2 to 20% (w/v), as follows.

More specifically, 20 mL of distilled water was poured into a transparent container at room temperature, and the aforementioned ICG-MAA complex was added thereto to measure a varying density of the distilled water including the ICG-MAA complex. In particular, the varying density according to the concentration of the ICG-MAA complex was measured.

For reference, it can be seen that, when the ICG-MAA complex was added and dissolved in distilled water, the distilled water exhibited white suspensibility.

Table 2 lists the densities with respect to distilled water including the ICG-MAA complexes, which are summarized according to the concentration of distilled water including the ICG-MAA complexes.

[Table 2]

| ICG-MAA complex (% (w/v)) | Density (g/mL) |
|---|---|
| 20 | 1.0616 |
| 12 | 1.0395 |
| 10 | 1.0338 |
| 8 | 1.0283 |
| 6 | 1.0228 |
| 4 | 1.0177 |
| 2 | 1.0118 |

Referring to Table 2, a linear regression equation was obtained through linear regression analysis, and the density when it reached 100% was measured by extrapolation (0.2757*100+1.0064 = 1.2821).

As a result, it was estimated that the density of the ICG-MAA complex was 1.2821 g/mL according to the regression equation (FIG. 1).

In addition, it can be seen that, when the distilled water in which the ICG-MAA complex was dissolved was checked after 20 minutes, a white suspension had settled on the bottom of the container.

As a result, it was judged that the density of the ICG-MAA complex was 1.2821 g/mL, which was higher than the density (0.99821 g/mL) of the distilled water at room temperature.

**Experimental** Example 2: Evaluation of usefulness of **injection composition**

**Experimental Example 2-1: Usefulness of ICG-MAA-HA as injection composition-1**

To evaluate the usefulness of ICG-MAA-HA as an injection composition, a suspensibility maintenance time of ICG-MAA-HA according to the concentration of HA was measured. For reference, MAA was used at a concentration of 2 mg/mL, and the final ICG-MAA-HA injection solution had a characteristic of a light green suspension when MAA and ICG were completely dissolved.

More specifically, the suspensibility maintenance time after preparation of the ICG-MAA-HA injection solution was measured, and the suspensibility maintenance time was measured according to an increasing concentration of HA. Here, the suspensibility defines a suspensibility maintenance time as a reference point of time when a settling region and a floating region are separated based on 20% transmittance of light with 500 nm.

The results are listed in Table 2.

Table 2 lists the suspensibility maintenance times after the preparation of the ICG-MAA-HA injection solution according to the concentration of the HA solvent.

[Table 3]

| Concentration (% (w/v)) of HA solvent | Suspensibility maintenance time |
|---|---|
| 0 % (simple water for injection) | Within 10 minutes |
| 0.1 % | Within 30 minutes |

(continued)

| Concentration (% (w/v)) of HA solvent | Suspensibility maintenance time |
|---|---|
| 0.2% | Within 2 hours |
| 0.3 % | Within 8 hours |
| 0.4 % | Within 14 hours |
| 0.5 % | Within 24 hours |
| * A dissolution time is shortened when the injection is strongly stirred using a mechanical stirrer, but the dissolution time is not constant according to stirring strength when the injection is gently stirred. | |

Referring to Table 3, it can be seen that the injection composition to which HA was not added had a shorter suspensibility maintenance time than the composition to which HA was added, and the suspensibility maintenance time was lengthened with an increasing proportion of added HA.

Meanwhile, it was confirmed that the suspensibility maintenance time increased to less than 30 minutes when 0.1% HA was added to the injection composition, and that the suspensibility maintenance time was greater than 2 hours when the concentration of HA was greater than 0.2% (w/v), and the suspensibility maintenance time was greater than 8 hours when the concentration of HA was greater than 0.3% (w/v).

That is, it was judged that the complex was actually effective as an injectable preparation when the concentration of HA was greater than 0.2% (w/v).

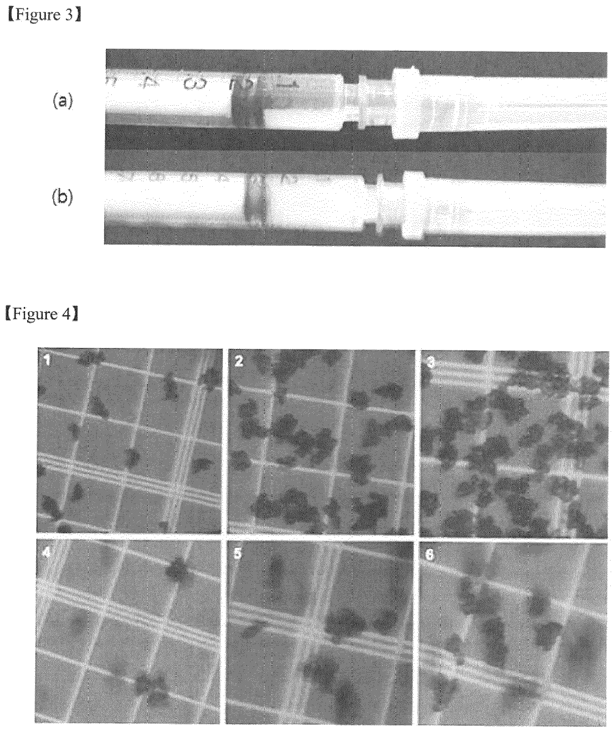

FIG. 3 shows an image illustrating a comparison between two types of MAA having different HA concentrations.

More specifically, as the images of the ICG-MAA-HA injection thus prepared photographed after 30 minutes, FIG. 3(A) is an image of MAA to which 0.1% HA is added, and FIG. 3(B) is an image of MAA to which 0.5% HA is added.

Referring to FIG. 3, it can be seen that the injection composition to which 0.1% HA was added settled rapidly, but the injection composition to which 0.5% HA was added did not settle for a long time. That is, it can be seen that because the ICG-MAA-HA in which 0.5% HA was added to ICG-MAA did not settle rapidly during injection preparation, it can be very useful as an injection composition, as shown in FIG. 3.

**Experimental Example 2-2: Usefulness of ICG-MAA-HA as injection composition-2**

In Experimental Example 2-2, gliding forces exerted on a finger upon injection according to the concentration of hyaluronic acid (HA) were measured under various conditions, and the usefulness of the ICG-MAA-HA as an injection composition was judged.

More specifically, when the finally prepared injection solution was used as an injection, the ease of injection was evaluated using Ice disposable syringes equipped with 18G, 21G, 22G, 23G, and 26G needles.

Meanwhile, for the characteristics of the composition, loads applied upon injection were examined using an 18-gauge needle (inner needle diameter: 0.838 mm), a 21G needle (inner needle diameter: 0.495 mm), a 22G needle (inner needle diameter: 0.394 mm), a 23G needle (inner needle diameter: 0.318 mm), and a 26G needle (inner needle diameter: 0.241 mm).

In addition, resistance (a force exerted on a finger upon injection) was measured using an endoscopic catheter (7 Fr, length: 180 cm, MTW, Germany) equipped with a 1.8 m-long connection tube.

The results are listed in the following Table 4.

[Table 4]

| Syringe | gf | Water | Hyaluronic acid (% (w/v)) | | | | | | | | | | | |
|---|---|---|---|---|---|---|---|---|---|---|---|---|---|---|---|
| | | | 0.10 | 0.20 | 0.30 | 0.40 | 0.50 | 0.60 | 0.70 | 0.80 | 0.90 | 1.00 | 2.00 | 3.00 |
| 26 G | 79 | 91 | 107.5 | 110.0 | 115.0 | 120.0 | 125.0 | 131.0 | 136.0 | 142.0 | 146.0 | 152.5 | 253.2 | Not injectable |
| 23 G | 82.6 | 81 | 97.5 | 100.0 | 102.0 | 104.0 | 107.0 | 112.0 | 120.0 | 128.0 | 132.0 | 138.0 | 240.6 | Not injectable |
| 22 G | 72.5 | 72.5 | 92.5 | 93.0 | 95.0 | 97.0 | 99.0 | 110.0 | 115.0 | 118.0 | 120.0 | 124.0 | 223.8 | 690.4 |
| 21 G | 67 | 72.5 | 87.5 | 93.0 | 90.0 | 91.0 | 92.5 | 97.0 | 102.0 | 105.0 | 110.0 | 114.0 | 220.5 | 650.2 |
| 18 G | 62 | 75 | 78 | 81.0 | 83.0 | 85.0 | 87.5 | 91.0 | 95.0 | 99.0 | 102.0 | 104.0 | 208.9 | 640.3 |
| 7Fr | | 145 | 280 | 380 | 490 | 600 | 631 | 780 | Not injectable | Not injectable | Not injectable | Not injectable | Not injectable | Not injectable |

14

Generally, when an injection needle used for clinical trials was used, a force of 60 to 140 gf was applied during injection. The injection needle was used without difficulty due to the low resistance during injections until the concentration of hyaluronic acid (HA) reached 1%.

On the other hand, when an endoscopic catheter needle (7Fr, 180 cm) in which resistance was increased due to a long tube was used, a force of 140 gf was applied in the case of water, and a force of 780 gf was applied in the case of HA at a concentration of 0.60% (w/v), indicating that the injection solution was injectable with a remarkably high pressure.

Although this may differ for individuals, when a gliding force of 700 gf was applied, it was difficult to inject the injection solution into tissues, which made it difficult to accurately inject the injection solution. On the other hand, when an endoscopic catheter needle (7Fr, 180 cm) was used, a force of 780 gf was applied when HA was present at a concentration of 0.60%, indicating that the injection solution was injectable with a remarkably high pressure. The injection solution was not injectable by hand due to high resistance when the concentration of HA was greater than 0.70%.

That is, the injection solution was injectable under general injection conditions until the concentration of hyaluronic acid reached 1% (w/v), and the injection solution was injectable by hand until the concentration of hyaluronic acid reached 0.5% (w/v) in the case of a specialty endoscopic catheter having a long needle, in which a syringe was connected to a needle via a tube.

Meanwhile, it can be seen that, when general syringes (26G to 18G) coming into direct contact with tissues were used, it was desirable for the injection solution to be smoothly injected into the tissues, that is, a force of less than 200 gf was applied to the syringes, and it was possible to inject the injection solution with a force of less than 200 gf until the concentration of hyaluronic acid reached 1% (w/v).

**Experimental Example 3: Change in syringe pressure according to precipitation of ICG-MAA-HA**

**Experimental Example 3-3: Change in syringe pressure according to precipitation of ICG-MAA-HA-1**

In Example 3-3, the pressure (A) applied to the syringe when the injection composition to which hyaluronic acid was added was injected immediately after the addition of HA and the pressure (B) applied to the syringe after the injection composition was allowed to stand for 120 minutes so that the composition settled were measured, and are listed in the following table.

In this case, the syringe equipped with a 26G needle was used to measure a gliding force of the syringe.

In addition, the unit of the gliding force is gf representing gravitational force (1 g = 0.0098 N).

[Table 5]

| HA (%) | 0 | 0.1 | 0.2 | 0.3 | 0.4 | 0.5 | 0.6 | 0.7 | 0.8 | 0.9 | 1.0 |
|---|---|---|---|---|---|---|---|---|---|---|---|
| A | 91 | 107.5 | 110.0 | 115.0 | 120.0 | 125.0 | 131.0 | 136.0 | 142.0 | 146.0 | 152.5 |
| B | 400 | 310 | 125 | 126 | 130 | 140 | 144 | 152 | 160 | 155 | 165 |
| B-A | 309 | 202.5 | 15 | 11 | 10 | 15 | 13 | 16 | 18 | 9 | 12.5 |

Referring to Table 5, it can be seen that the pressure (A) applied to the syringe when the injection composition to which hyaluronic acid was added was injected immediately after the addition of HA and the pressure (B) applied to the syringe after the injection composition was allowed to stand for 120 minutes so that the composition settled increased with an increasing concentration of hyaluronic acid.

In addition, as the concentration of hyaluronic acid increased, there was no big difference between the pressure (B) applied to the syringe after the injection composition was allowed to stand for 120 minutes and the pressure (A) applied to the syringe when the injection composition was injected immediately. Particularly, from when the concentration of hyaluronic acid increased from 0.2% (w/v) to 1.0% (w/v), an average B-A value was 13.27778 gf, and the standard deviation was 2.969755.

**Experimental Example 3-3: Change in syringe pressure (gliding force) according to precipitation of ICG-MAA-HA-2**

Generally, a pressure applied to the syringe with a 26G needle according to the concentration of hyaluronic acid increased from approximately 20 gf (0.1% HA) to 70 gf (1.0% HA). The pressure applied to the syringe increased according to an injection composition other than hyaluronic acid in the injection. In one example, a force of approximately 15 gf was further applied when MAA was present at a concentration of 8 mg/mL.

Therefore, in Experimental Example 3-3, the pressures applied to the syringe when the injection composition to which hyaluronic acid was added was injected immediately after addition of hyaluronic acid and after the injection composition was allowed to stand for 120 minutes to allow the composition to settle were measured, and are listed in the following table.

For reference, in Table 6, (A) lists the pressures when the injection composition was injected immediately, and (B) lists the pressures at the start of injection after the injection composition was allowed to stand for 120 minutes. Also, (C) lists the pressures (forces exerted on a finger, i.e., gliding forces) at an intermediate point of injection after the injection composition was allowed to stand for 120 minutes.

[Table 6]

| HA (%) | 0 | 0.1 | 0.2 | 0.3 | 0.4 | 0.5 | 0.6 | 0.7 | 0.8 | 0.9 | 1.0 | 2.0 | 3.0 |
|---|---|---|---|---|---|---|---|---|---|---|---|---|---|
| A | 115 | 120 | 123 | 127.5 | 132.5 | 137.5 | 143.5 | 148.5 | 154.5 | 158.5 | 165 | 252 | 721 |
| B | 400 | 310 | 125 | 126 | 130 | 140 | 144 | 152 | 160 | 155 | 165 | 257 | 711 |
| C | 110 | 120 | 125 | 128 | 138 | 138 | 142 | 150 | 155 | 160 | 170 | 253 | 718 |

Referring to Table 6, it can be seen that, when hyaluronic acid was not added, the pressure was greater than 400 gf when the injection was started after the injection composition was allowed to stand for 2 hours, indicating that the injection needle was clogged with the composition in the injection.

Also, the pressure dropped to 120 gf as the needle clogged with the composition was opened up.

When hyaluronic acid was present at a concentration of 0.2% (w/v) or more, the injection needle was not clogged with the composition in the injection even after the composition was allowed to stand for 120 minutes, and thus the composition was injectable with a small force (200 gf) like that when the composition was injected immediately.

That is, when hyaluronic acid was added at a proper amount (0.2% or more), the precipitation rate was able to be slowed down, and the drawback of having to apply an excessive force to start injection due to the injection needle being clogged with the precipitating composition was able to be solved.

Meanwhile, when the concentration of hyaluronic acid was greater than 1% (w/v), the resistance during injection was high due to the viscosity of hyaluronic acid.

**Experimental Example 3: Change in transmittance according to precipitation of ICG-MAA-HA**

A change in optical density over time was measured for the injection composition prepared in Example 1. For reference, a change in optical density was measured for an injection composition including 2 mg/mL of MAA.

In addition, transmittance was obtained by measuring the transmittance of visible light using a UV spectrometer (TECAN. Infinite M200PRO).

More specifically, when transmittance was measured at the one quarter point of the height of a transparent container having a size of $1 \times 1 \times 3$ cm$^3$, using a UV spectrometer, in a state in which the injection composition was loaded in the container, transmittance at 550 nm was measured.

In this case, $T_1$ represents transmittance (%) at 550 nm when measured at the one quarter point of the height of a transparent container having a size of $1 \times 1 \times 3$ cm$^3$ in a state in which the prepared injection composition is loaded in the transparent container, and $T_2$ represents transmittance (%) at 550 nm when measured at the one quarter point of the height of a transparent container having a size of $1 \times 1 \times 3$ cm$^3$ after being allowed to stand for 120 minutes in a state in which the prepared injection composition is loaded in the transparent container.

[Table 7]

| HA (%) | 0.0 | 0.2 | 0.4 | 0.6 | 0.8 | 1.0 | 1.2 | 1.4 | 1.6 | 1.8 | 2.0 |
|---|---|---|---|---|---|---|---|---|---|---|---|
| $T_1$ | 77.76 | 78.76 | 78.53 | 78.61 | 78.78 | 78.65 | 78.48 | 78.18 | 77.97 | 77.69 | 77.65 |
| $T_2$ | 87.88 | 87.95 | 78.71 | 78.77 | 78.65 | 78.71 | 78.56 | 78.20 | 78.11 | 77.81 | 77.73 |
| $|T_2-T_1|$ | 10.12 | 9.19 | 0.18 | 0.16 | 0.13 | 0.06 | 0.08 | 0.02 | 0.14 | 0.12 | 0.08 |

Referring to Table 7, it was confirmed that, when the change in transmittance according to the precipitation of ICG-MAA-HA was examined, the transmittance measured at the beginning when hyaluronic acid was not added was 77.76%, and the transmittance increased to 87.88% when the injection composition was allowed to stand for 120 minutes.

This was because the injection composition was first suspended as the ICG-MAA was dissolved in the injection composition, and the ICG-MAA particles settled due to high density after 120 minutes, resulting in increased transmittance at the one quarter point of the container from the bottom thereof.

In addition, when the concentration of hyaluronic acid (HA) was greater than or equal to 0.4% (w/v), a difference between the transmittance ($T_1$) measured at the beginning and the transmittance ($T_2$) measured after the injection composition was allowed to stand for 120 minutes was insignificant at less than 1%.

This indicates that the suspensibility of the injection composition was maintained constant over time when the ICG-MAA-HA was included in the injection composition.

As a result, it can be seen that the complex was able to be prevented from settling rapidly in the injection composition when the viscosity of the ICG-MAA complex was controlled by adding hyaluronic acid (HA) to the injection composition.

Meanwhile, it can be seen that the complex settled within 2 hours because there was a difference of 9.19% between $T_1$ and $T_2$ when hyaluronic acid was present at a concentration of 0.2% (w/v). However, it was judged that the ICG-MAA complex would be able to be used as an injection composition even when 0.2% hyaluronic acid (HA) was added to the ICG-MAA complex because the ICG-MAA complex is typically administered within 30 minutes when prepared for use as an injection composition.

**Experimental Example 4: Bright-field image of ICG-MAA according to application of HA**

FIG. 4 shows a bright-field image of ICG-MAA-HA injection solutions including 0.5% (w/v) HA, 6.5 $\mu$M ICG, and 2, 4, and 8 mg/mL of MAA and ICG-MAA injection solutions including 6.5 $\mu$M ICG and 2, 4, and 8 mg/mL of MAA. An MAA particle distribution was visualized using an optical microscope with a hemocytometer. Each square represents a size of 0.05 $mm^2$. An upper panel (FIG. 4-1, 2 and 3) shows ICG-MAA including no HA, and a lower panel (FIG. 4-4, 5 and 6) shows ICG-MAA-HA including 0.5 % HA (w/v). The concentrations of MAA are as follows: FIG. 4-1 and 4-4: 2 mg/mL; FIG. 4-2 and 4-5: 4 mg/mL; and FIG. 4-3 and 4-6: 8 mg/mL. In the upper panel, a clear image was obtained because the MAA particles were in focus in a bright field as all the MAA particles had settled. On the other hand, in the lower panel, because the MAA particles settled and floated in the injection solutions, some particles were in focus and a clear image of the particles was obtained, and other particles were not in focus in the image.

**Experimental Example 5: Fluorescence maintenance characteristics in ICG-MAA-HA**

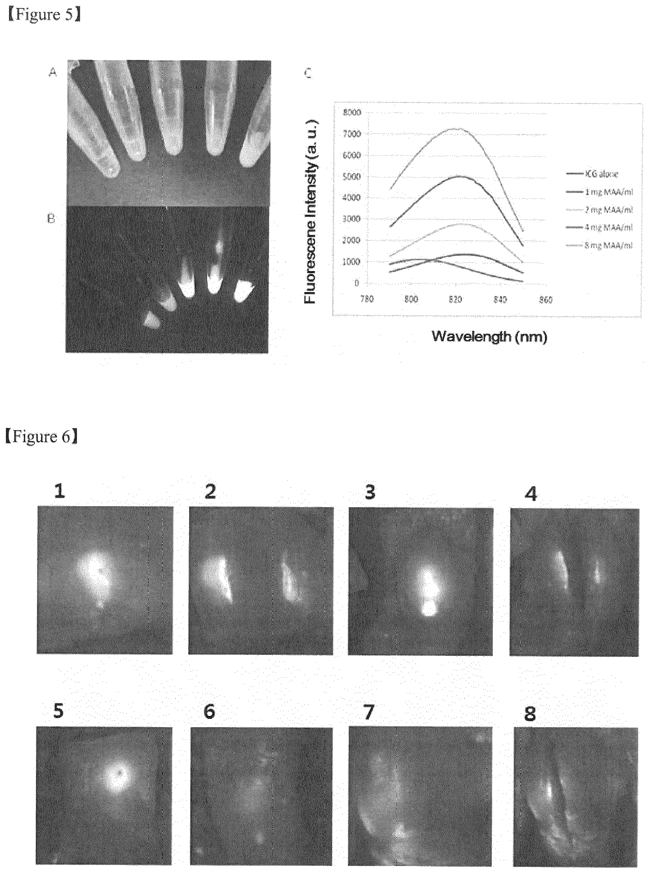

FIG. 5 shows fluorescence signals measured for ICG-MAA-HA, to which 5 $\mu$M ICG and 0.5% HA are added, at various concentrations of MAA. FIG. 5A shows a bright-field image, and FIG. 5B shows an NIRF image. The concentrations of MAA were 0 mg/mL, 1 mg/mL, 2 mg/mL, 4 mg/mL, and 8 mg/mL, as shown from left to right in FIGS. 5A and 5B. The NIRF image was obtained by emitting light from 17 2mA NIR LEDs having a peak wavelength of 740 nm. The image was photographed and visualized by AVT UniCam viewer software (Allied Vision Technologies) according to the following settings of a camera: Exposure time: 200 ms; Gain: 200; Target grayscale value: 125; and Brightness: 16.

Because the MAA present in water had settled very rapidly onto walls of Eppendorf tubes, the present inventors photographed a bright-field image, mixed the respective tubes, and then repositioned the tubes. In this case, small changes in positions of the tubes were observed between FIG. 5A and 5B.

FIG. 5C shows the fluorescence emission spectra of 5 $\mu$M ICG present at various concentrations of MAA. The relative fluorescence intensity (a.u: arbitrary unit) was obtained using a computer-controlled fluorescence microplate reader (Safire II; Tecan, Durham, NC). The excitation wavelength for ICG was 760 nm, and the emission wavelength was in a range of 790 to 850 nm.

That is, it can be seen that the fluorescence was maintained for ICG-MAA-HA, as shown in FIGS. 5A to 5C.

**Experimental Example 6: Results of injecting ICG-MAA-HA into chicken breast**

FIG. 6 shows the experimental results illustrating a difference according to addition of 0.5% (w/v) HA. Also, a difference in tissue strength was determined using a chicken breast having typical tissue strength and a chicken gizzard having compact tissue strength. 50 $\mu$L of each of ICG-MAA-HA and ICG-MAA was slowly injected 5 times into the chicken breast and chicken gizzard to a depth of 5 mm. An NIRF image was obtained by emitting light from 17 2mA NIR LEDs having a peak wavelength of 740 nm. The image was photographed and visualized by AVT UniCam viewer software (Allied Vision Technologies) according to the following settings of a camera: Exposure time: 195 ms; Gain: 170; Target grayscale value: 125; and Brightness: 16. It took approximately 2 to 3 minutes to prepare and inject the injections. When the ICG-MAA was injected twice out of the five times into the chicken breast and injected three out of the five times into the chicken gizzard, the ICG-MAA particles had settled and clogged the injection needle, which made it difficult to inject the injection solutions. When it was difficult to inject the injection solutions, the syringe was shaken, and the injection solutions were again injected within a short time. On the other hand, the ICG-MAA-HA was always injected smoothly over ten attempts.

FIGS. 6-1 and 6-2 are NIRF images photographed before and after incision after the ICG-MAA-HA including 0.5% HA was injected into the chicken breast. It can be seen that the labels were properly distributed. FIGS. 6-3 and 6-4 are NIRF images photographed before and after the incision after the ICG-MAA-HA including 0.5% HA was injected into the chicken gizzard. Also, it can be seen that the labels were properly distributed. FIGS. 6-5 and 6-6 are two NIRF images photographed after the ICG-MAA including no 0.5% HA was injected into the chicken breast. It was judged that the labels were properly distributed, as shown in FIG. 6-5, but a small amount of the labels were injected because the ICG-MAA particles had settled during injection when injected at the same total amount, as shown in FIG. 6-6. FIGS. 6-7 and 6-8 are NIRF images photographed before and after the incision after the ICG-MAA including no 0.5% HA is injected into the chicken gizzard. The ICG-MAA flowed backward along an injection track so that the labels were not properly distributed.

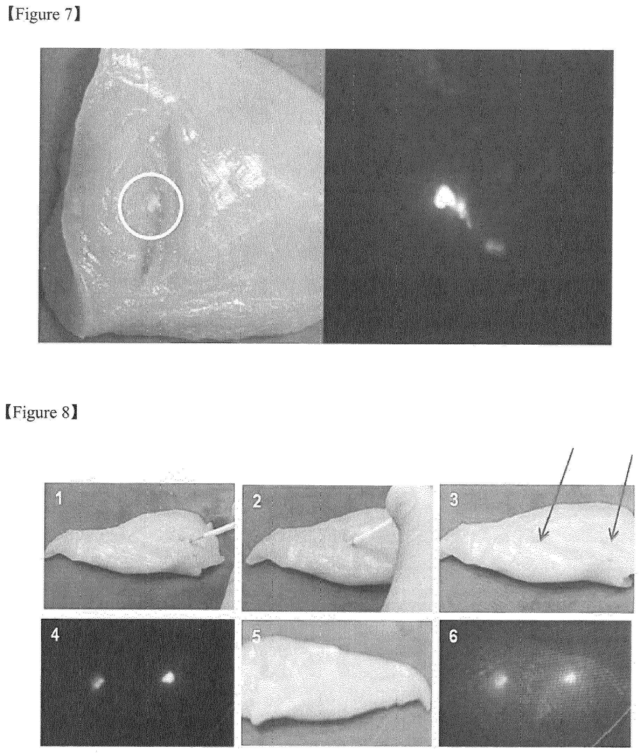

FIG. 7 shows a bright-field and NIRF image of a section of an incised chicken breast visualized after the complex was injected along an injection needle pathway. A region into which the ICG-MAA-HA was injected is indicated by a white circle on the bright-field image. The NIRF image was obtained by emitting light from the 17 2mA NIR LEDs having a peak wavelength of 740 nm. The image was photographed and visualized by AVT UniCam viewer software (Allied Vision Technologies) according to the following settings of a camera: Exposure time: 195 ms; Gain: 170; Target grayscale value: 125; and Brightness: 16.

From the results, it can be seen that, when the ICG-MAA-HA was injected into the tissues (chicken breast), the fluorescence characteristics were maintained well, and the spreadability was also very low. That is, it can be seen that the problem of the ICG-MAA complex spreading into the surroundings too fast and too far was able to be solved using the ICG-MAA-HA.

**Experimental Example 7: Results of injecting ICG-MAA-HA into chicken breast using endoscopic catheter**

FIG. 8 shows the testing of the applicability of an ICG-MAA-HA mixture in gastric cancer surgery using an endoscopic catheter. FIGS. 8-1 to 8-3 and 8-5 show bright-field images, and FIGS. 8-4 and 8-6 show NIRF images:

FIG. 8-1: An ICG-MAA-HA mixture [6.5 $\mu$M ICG, 2 mg/mL MAA, and 0.5% HA (w/v)] was injected into one side of the chicken breast using a disposable endoscopic catheter;
FIG. 8-2: The mixture was injected into a second site of the chicken breast;
FIG. 8-3: Two arrows represent the sites for injections;
FIG. 8-4: shows an NIRF image of sites for injections like those lining the walls of the stomach;
FIG. 8-5: shows an image of an inverted injected chicken breast;
FIG. 8-6: shows an NIRF image of sites for injections on the inverted chicken breast. In this case, the sites for injections can be seen using external NIR sources.

The NIRF images were obtained by emitting light from the 17 2mA NIR LEDs having a peak wavelength of 740 nm. The image was photographed and visualized by AVT UniCam viewer software (Allied Vision Technologies) according to the following settings of a camera: Exposure time: 195 ms; Gain: 170; Target grayscale value: 125; and Brightness: 16.

From the results, it can be seen that, when the ICG-MAA-HA was injected into the tissues (chicken breast) using an endoscopic catheter, the fluorescence characteristics were maintained well, and the spreadability was also very low. That is, it can be seen that, even when the surgery for gastric cancer was carried out as with the chicken breast, the ICG-MAA complex was able to be prevented from spreading to the surroundings too fast and too far while maintaining the fluorescence characteristics using the ICG-MAA-HA.

**Experimental Example 8: Constant concentration effect of ICG-MAA-HA**

To examine an effect of injecting a predetermined amount of the injection composition according to an increase in precipitation rate, each of the ICG-MAA injection composition to which hyaluronic acid was not added and the ICG-MAA-HA injection composition to which 0.1% (w/v) HA was added was sequentially injected into a tube at an amount of 100 $\mu$L to measure a fluorescence signal.

Meanwhile, the fluorescence signals were measured after the injection composition was injected into the tubes and precipitated for 5 minutes.

For reference, when the MAA was present at a concentration of 2 mg/mL, 1.5 $\mu$M (approximately 0.001 mg) ICG was added to the ICG-MAA injection composition, and 0.1% (w/v) HA was added to the ICG-MAA injection composition.

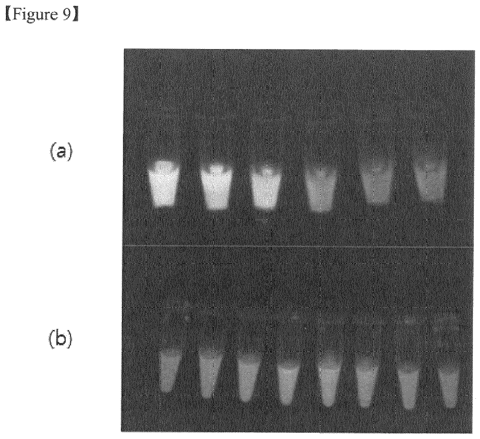

FIG. 9 is a diagram showing the results of measuring fluorescence signals according to the presence/absence of hyaluronic acid (at an initial amount of 100 $\mu$L from the left: (A) hyaluronic acid is not added, and (B) 0.1% hyaluronic acid is added).

The near-infrared fluorescence (NIRF) images of FIG. 9 were obtained by emitting light from the 17 2mA NIR LEDs having a peak wavelength of 740 nm. The images were photographed and visualized by AVT UniCam viewer software (Allied Vision Technologies) according to the following settings of a camera: Exposure time: 200 ms; Gain: 200; Target grayscale value: 125; and Brightness: 16.

Referring to FIG. 9, the CV (standard deviation/mean) of the volume of the injected composition was 28% in the case of the injection composition to which hyaluronic acid was not added.

In particular, when the fluorescence intensities were compared as shown in FIG. 9(A), there was a big difference in fluorescence intensities between the left and right. From the results, it was judged that a large amount of the ICG-MAA was injected at the beginning, but a decreasing amount of the ICG-MAA was injected thereafter, indicating that the predetermined amount of ICG-MAA was not injected.

On the other hand, referring to FIG. 9(B), it can be seen that, when hyaluronic acid was added to the injection composition, the settling of the ICG-MAA had slowed down, thereby allowing the predetermined amount of ICG-MAA to be injected.

The present invention has been described in detail. However, it should be understood by persons having ordinary skill in the art to which the present invention belongs that the detailed description and specific examples, while indicating preferred embodiments of the invention, are given by way of illustration only.

**Claims**

1. An injection composition for labeling a lesion, comprising:

    a first composition comprising a complex containing an active ingredient and having an average density of 1.1 to 1.4 g/mL, the active ingredient being a dye for staining biological tissues, a radioactive isotope or a combination thereof bound to macroaggregated albumin (MAA); and
    a second composition being hyaluronic acid (HA), the HA having an average molecular weight of 0.5 to 3.0 MDa, wherein the HA is included at 0.2 to 1 % (w/v) with respect to the total amount of the injection composition.

2. The injection composition of claim 1, wherein the injection composition satisfies the following Mathematical Expression 1 when measured at room temperature:

$$[\text{Mathematical Expression 1}]$$
$$|T_2 - T_1| < 15\%$$

    wherein $T_1$ represents transmittance (%) at 550 nm when measured at the one quarter point of the height of a transparent container having a size of $1 \times 1 \times 3$ cm$^3$ in a state in which the prepared injection composition is loaded in the transparent container, and $T_2$ represents transmittance (%) at 550 nm when measured at the one quarter point of the height of a transparent container having a size of $1 \times 1 \times 3$ cm$^3$ after being allowed to stand for 120 minutes in a state in which the prepared injection composition is loaded in the transparent container.

3. The injection composition of claim 1, wherein the injection composition satisfies the following Mathematical Expression 2 when measured at room temperature:

$$[\text{Mathematical Expression 2}]$$
$$|F_2 - F_1| < 20 \text{ gf}$$

    wherein $F_1$ represents a gliding force measured at the start of injection in a state in which the prepared injection composition is loaded in a syringe with a 26G needle, and
    $F_2$ represents a gliding force measured at the start of injection when injected after being allowed to stand for 120 minutes in a state in which the prepared injection composition is loaded in a syringe with a 26G needle.

4. The injection composition of claim 3, wherein the gliding force measured according to Mathematical Expression 2 is less than or equal to 15 gf.

5. The injection composition of claim 3, wherein the $F_2$ has a gliding force of 120 to 165 gf.

6. The injection composition of claim 1, wherein the dye for staining biological tissues is a visually detectable dye or a fluorescent dye, and
the radioactive isotope is selected from the group consisting of H-3, C-14, P-32, S-35, Cl-36, Cr-51, Co-57, Co-58, Cu-64, Fe-59, Y-90, I-124, I-125, Re-186, I-131, Tc-99m, Mo-99, P-32, Cr-51, Ca-45, Ca-68, and a combination thereof.

7. A method for providing information on a position of a lesion to which the injection composition of claim 1 has been administered, comprising:
determining the position of the lesion through a signal of an active ingredient generated in the subject.

**Patentansprüche**

1. Injektionszusammensetzung zum Markieren einer Läsion, umfassend:

eine erste Zusammensetzung, die einen Komplex umfasst, der einen Wirkstoff enthält und eine durchschnittliche Dichte von 1,1 bis 1,4 g/ml aufweist, wobei der Wirkstoff ein Farbstoff zum Färben biologischer Gewebe, ein radioaktives Isotop oder eine Kombination davon, gebunden an makroaggregiertes Albumin (MAA), ist; und eine zweite Zusammensetzung, die Hyaluronsäure (HA) ist, wobei die HA ein durchschnittliches Molekulargewicht von 0,5 bis 3,0 MDa aufweist, wobei die HA zu 0,2 bis 1% (Gew./Vol.) bezogen auf die Gesamtmenge der Injektionszusammensetzung umfasst ist.

2. Injektionszusammensetzung nach Anspruch 1, wobei die Injektionszusammensetzung den folgenden mathematischen Ausdruck 1 erfüllt, wenn sie bei Raumtemperatur gemessen wird:

$$[\text{Mathematischer Ausdruck 1}]$$
$$|T_2\text{-}T_1| < 15\%$$

wobei $T_1$ die Transmission (%) bei 550 nm darstellt, gemessen am Viertelpunkt der Höhe eines transparenten Behälters mit einer Größe von 1x1x3 cm$^3$ in einem Zustand, in dem die hergestellte Injektionszusammensetzung in den transparenten Behälter geladen ist, und
$T_2$ die Transmission (%) bei 550 nm darstellt, gemessen am Viertelpunkt der Höhe eines transparenten Behälters mit einer Größe von 1x1x3 cm$^3$, nachdem er 120 Minuten lang stehen gelassen wurde, in einem Zustand, in dem die hergestellte Injektionszusammensetzung in den transparenten Behälter geladen ist.

3. Injektionszusammensetzung nach Anspruch 1, wobei die Injektionszusammensetzung den folgenden mathematischen Ausdruck 2 erfüllt, wenn sie bei Raumtemperatur gemessen wird:

$$[\text{Mathematischer Ausdruck 2}]$$
$$|F_2\text{-}F_1| < 20 \text{ gf}$$

wobei $F_1$ eine Gleitkraft darstellt, die zu Beginn der Injektion gemessen wird, in einem Zustand, in dem die hergestellte Injektionszusammensetzung in eine Spritze mit einer 26G-Nadel geladen ist, und
$F_2$ eine Gleitkraft darstellt, die zu Beginn der Injektion gemessen wird, wenn die Injektion erfolgt, nachdem sie 120 Minuten lang stehen gelassen wurde, in einem Zustand, in dem die hergestellte Injektionszusammensetzung in eine Spritze mit einer 26G-Nadel geladen ist.

4. Injektionszusammensetzung nach Anspruch 3, wobei die gemäß dem mathematischen Ausdruck 2 gemessene Gleitkraft kleiner oder gleich 15 gf ist.

5. Injektionszusammensetzung nach Anspruch 3, wobei $F_2$ eine Gleitkraft von 120 bis 165 gf aufweist.

6. Injektionszusammensetzung nach Anspruch 1, wobei der Farbstoff zum Färben biologischer Gewebe ein visuell nachweisbarer Farbstoff oder ein Fluoreszenzfarbstoff ist, und
das radioaktive Isotop ausgewählt ist aus der Gruppe bestehend aus H-3, C-14, P-32, S-35, Cl-36, Cr-51, Co-57, Co-58, Cu-64, Fe-59, Y-90, I-124, I-125, Re-186, I-131, Tc-99m, Mo-99, P-32, Cr-51, Ca-45, Ca-68 und einer Kombination davon.

7. Verfahren zur Bereitstellung von Informationen bezüglich einer Position einer Läsion, der die Injektionszusammensetzung nach Anspruch 1 verabreicht wurde, umfassend:
Bestimmen der Position der Läsion durch ein Signal eines Wirkstoffs, das in dem Individuum erzeugt wurde.

**Revendications**

1. Composition d'injection pour marquer une lésion, comprenant :

une première composition comprenant un complexe contenant un ingrédient actif et ayant une densité moyenne comprise entre 1,1 et 1,4 g/mL, l'ingrédient actif étant un colorant pour colorer des tissus biologiques, un isotope radioactif ou une combinaison de ceux-ci lié(e) à de l'albumine macro-agrégée (MAA) ; et
une seconde composition étant de l'acide hyaluronique (HA), l'HA ayant un poids moléculaire moyen comprise entre 0,5 et 3,0 MDa, dans laquelle l'HA est inclus entre 0,2 et 1 % (p/v) par rapport à la quantité totale de la composition d'injection.

2. Composition d'injection selon la revendication 1, dans laquelle la composition d'injection satisfait à l'expression mathématique 1 suivante lorsqu'elle est mesurée à température ambiante :

[Expression mathématique 1]

$$|T_2 - T_1| < 15\ \%$$

dans laquelle $T_1$ représente la transmittance (%) à 550 nm lorsqu'elle est mesurée au niveau du quart de point de la hauteur d'un récipient transparent ayant une taille de $1 \times 1 \times 3$ cm$^3$ dans un état dans lequel la composition d'injection préparée est chargée dans le récipient transparent, et $T_2$ représente la transmittance (%) à 550 nm lorsqu'elle est mesurée au niveau du quart de point de la hauteur d'un récipient transparent ayant une taille de $1 \times 1 \times 3$ cm$^3$ après avoir été laissée au repos pendant 120 minutes dans un état dans lequel la composition d'injection préparée est chargée dans le récipient transparent.

3. Composition d'injection selon la revendication 1, dans laquelle la composition d'injection satisfait à l'expression mathématique 2 suivante lorsqu'elle est mesurée à température ambiante :

[Expression mathématique 2]

$$|F_2 - F_1| < 20\ \text{gf}$$

dans laquelle $F_1$ représente une force de glissement mesurée au début de l'injection dans un état dans lequel la composition d'injection préparée est chargée dans une seringue avec une aiguille de 26 G, et
$F_2$ représente une force de glissement mesurée au début de l'injection lorsqu'elle est injectée après avoir été laissée au repos pendant 120 minutes dans un état dans lequel la composition d'injection préparée est chargée dans une seringue avec une aiguille de 26 G.

4. Composition d'injection selon la revendication 3, dans laquelle la force de glissement mesurée selon l'expression mathématique 2 est inférieure ou égale à 15 gf.

5. Composition d'injection selon la revendication 3, dans laquelle le $F_2$ a une force de glissement comprise entre 120 et 165 gf.

6. Composition d'injection selon la revendication 1, dans laquelle le colorant pour colorer des tissus biologiques est un colorant détectable visuellement ou un colorant fluorescent, et
l'isotope radioactif est choisi dans le groupe constitué de H-3, C-14, P-32, S-35, Cl-36, Cr-51, Co-57, Co-58, Cu-64, Fe-59, Y-90, 1-124, 1-125, Re-186, 1-131, Tc-99m, Mo-99, P-32, Cr-51, Ca-45, Ca-68, et une combinaison de ceux-ci.

7. Procédé pour fournir des informations sur une position d'une lésion à laquelle la composition d'injection de la revendication 1 a été administrée, comprenant l'étape consistant à :
déterminer la position de la lésion par un signal d'un principe actif généré dans le sujet.

【Figure 1】

【Figure 2】

【Figure 3】

(a)

(b)

【Figure 4】

【Figure 5】

【Figure 6】

【Figure 7】

【Figure 8】

【Figure 9】

(a)

(b)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 101552138

- KR 2011009271 W

**Non-patent literature cited in the description**

- **T. KITAI et al.** *Breast Cancer,* 2005, vol. 12, 211-215